# FASCICULE DE BREVET EUROPEEN

(11) **EP 0 494 816 B1**
(45) Date de publication et mention de la délivrance du brevet: **21.07.1999**
(21) Numéro de dépôt: 92400030.0
(22) Date de dépôt: 07.01.1992
(51) Int. Cl.: C07D 211/90, C07D 401/12, C07D 409/14, A61K 31/44

(54) **Dérivés de 1,4-dihydropyridine, leur procédé de préparation et composition thérapeutique les contenant**
1,4-Dihydropyridin-Derivate, Verfahren zu ihrer Herstellung und diese enthaltende therapeutische Zusammensetzung
1,4-Dihydropyridine derivatives, process for their preparation and therapeutical composition containing them

(30) Priorité: 09.01.1991 FR 9100201
(43) Date de publication de la demande: 15.07.1992
(73) Titulaire: LABORATOIRE L. LAFON, 94701 Maisons Alfort (FR)
(72) Inventeur: Lafon, Louis, F 75016 Paris (FR)
(74) Mandataire: Le Guen, Gérard

(56) Documents cités:
- EP-A- 0 106 275
- EP-A- 0 214 094
- EP-A- 0 266 922
- DE-A- 3 303 066
- GB-A- 2 014 134
- PATENT ABSTRACTS OF JAPAN vol. 9, no. 233 (C-304)(1956) 19 Septembre 1985

## Description

La présente invention concerne de nouveaux dérivés de la 1,4-dihydropyridine.

Différents dérivés de 1,4-dihydropyridine ont déjà été décrits. Ainsi, notamment, dans FR-A-2 218 107 on a déjà décrit différents esters de l'acide 2,6-diméthyl-4-(3-nitrophényl)-5-méthoxycarbonyl-1,4-dihydropyridine-3-carboxylique et notamment l'ester 2[méthyl(phénylméthyl)amino] éthylique connu sous le nom de nicardipine.

La nicardipine est connue comme inhibiteur calcique et est utilisé en thérapeutique comme vasodilatateur et antihypertenseur.

D'autres 1,4-dihydropyridines à effet antihypertenseur sont décrites dans GB-A-2 014 134, EP-A-0 106 275, EP-A-0 266 922, DE-A-3 303 066, EP-A-0 214 094 et JP-A-60 092 266.

La présente invention vise à fournir de nouveaux inhibiteurs calciques qui présentent un effet antihypertenseur important et prolongé.

La présente invention a ainsi pour objet un composé qui est le 2,6-diméthyl-4-(3-nitrophényl)-5-méthoxycarbonyl-1,4-dihydropyridine-3-carboxylate de N[3-(2,4,6-triméthoxybenzoyl)propyl] pipéridine-3'-yle, et ses sels d'addition avec des acides pharmaceutiquement acceptables.

L'invention couvre non seulement les racémiques mais également l'ensemble des stéréoisomères associés à la présence de carbone asymétrique et éventuellement à l'empêchement de rotation de certaines liaisons.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; des sels métalliques acides, tels que l'orthophosphate disodique et le sulfate monopotassique, et des acides organiques.

Les exemples ci-après illustrent la préparation des composés selon l'invention.

### EXEMPLE1

### Préparation du 2,6-diméthyl-4-(3-nitrophényl)-5-méthoxycarbonyl-1,4-dihydropyridine-3-carboxylate de N-[3-(2,4,6, triméthoxybenzoyl)propyl]pipéridine-3'-yle, chlorhydrate. (CRL 41695)

### a) Préparation de l'acétoacétate de 2-cyanoéthyle.

On chauffe vers 90-100° C pendant 4 h 30 un mélange de 16,33 g (0,23 mole) de 3-hydroxy-propionitrile et de 28,4 g (0,20 mole) de 2,2,6-triméthyl-1,3-dioxen-4-one en présence de 0,1 ml de triéthylamine.

Le milieu réactionnel est purifié par une distillation sous pression réduite, pour donner 23,3 g d'une huile légèrement jaune.
E₄ₘₘ = 154 - 156°C.
Rendement = 75,2 %.

### b) Préparation du 2,6-diméthyl-4-(3-nitrophényl)-5-méthoxycarbonyl-1,4-dihydropyridine-3-carboxylate de 2-cyanoéthyle.

On porte au reflux pendant 20 heures sous atmosphère d'azote, un mélange de 54 g (0,348 mole) du produit obtenu en a), de 40 g (0,348 mole) de 3-aminocrotonate de méthyle et de 52,6 g (0,348 mole) de 3-nitro-benzaldéhyde dans 500 ml d'éthanol.

On traite le milieu réactionnel chaud au noir CXA, on concentre au 2/3 et après refroidissement, on isole par filtration 101,5 g d'une poudre jaune.
F.ᵢₙₛₜ (Kofler) = 126° C.
Rendement = 75,8 %.

### c) Préparation de l'acide 2,6-diméthyl-4-(3-nitrophényl)-5-méthoxycarbonyl-1,4-dihydropyridine-3-carboxylique.

On agite 2 heures à la température ambiante, une solution de 19,25 g (0,05 mole) du produit obtenu en b) et de 6 g (0,05 mole) de soude en pastilles dans 75 ml de 1,2-diméthoxy-éthane et 150 ml d'eau. On dilue le milieu réactionnel par de l'eau que l'on lave par du chlorure de méthylène, la phase organique est ensuite traitée par de l'acide chlorhydrique dilué et le précipité isolé par filtration.

Ce produit est purifié par un lavage à chaud dans l'éthanol, pour donner 14 g d'une poudre blanche.
F = 260° C.
Rendement = 84,34 %.

### d) Préparation du 1-(2,4,6-triméthoxybenzoyl)-3-chloropropane.

Au sein d'une solution maintenue vers +5° C de 67,2 g (0,400 mole) de 1,3,5-triméthoxy-benzène et de 61 g (0,432 mole) de chlorure de 4-chloro-butyryle dans 300 ml de benzène, on coule en 3 h 30 mn une solution de 121 g (0,464 mole) de tétrachlorure d'étain dans 150 ml de benzène. On agite 4 heures à la température ambiante et on jette le milieu réactionnel sur 500 ml d'eau glacée et 100 ml d'acide chlorhydrique 12 N. On décante la phase organique, que l'on lave par de l'eau et que l'on sèche sur sulfate de sodium sec.

Le résidu huileux est lavé avec de l'hexane, pour donner 97,2 g d'une poudre légèrement grise.
F._{inst.} (Kofler) = 48° C.
Rendement = 89,2 %.

### e) Préparation du 1-(2,4,6-triméthoxybenzoyl)-3-(3-hydroxypipéridino)propane, chlorhydrate.

Au sein d'une solution au reflux de 30 g (0,297 mole) de 3-hydroxy-pipéridine dans 80 ml de toluène, on coule en 1 h, une solution de 40,5 g (0,148 mole) du produit obtenu à l'exemple 1 d dans 30 ml de toluène. On poursuit le reflux 3 h, on dilue le milieu réactionnel par de l'acétate d'éthyle, on élimine l'insoluble par filtration et on extrait le filtrat par une solution d'acide chlorhydrique dilué. La phase aqueuse est alcalinisée par de la soude, extraite à son tour par de l'acétate d'éthyle qui, après séchage sur sulfate de sodium sec est traité par de l'isopropanol chlorhydrique.

Le précipité obtenu par filtration est purifié par une cristallisation dans de l'éthanol absolu pour donner 28 g d'une poudre blanche.
F. _{inst.} (Kofler) = 175° C.
Rendement = 50,7 %.

### f) Préparation du 2,6-diméthyl-4-(3-nitrophényl)-5-méthoxycarbonyl-1,4-dihydropyridine-3-carboxylate de N-[3-(2,4,6-triméthoxybenzoyl)propyl] pipéridine-3'-yle, chlorhydrate.

Au sein d'une solution maintenue à -25° C de 66 ml de diméthylformamide et de 38,5 ml d'acétonitrile, on coule en 15 mn une solution de 9 g (0,070 mole) de chlorure d'oxalyle dans 25 ml d'acétonitrile, on agite 15 mn à -25° C et on introduit : 16,75 g (0,050 mole) du produit obtenu à l'exemple 1 c. Après 1 heure à -15° C, on refroidit à -25° C et on coule une solution de 17 g (0,030 mole) du produit obtenu à l'exemple 1e (sous forme de base) dans 52,5 ml de pyridine et 11 ml de diméthylformamide. On agite 20 heures à la température ambiante, on jette le milieu réactionnel sur 500 ml d'eau glacée et 75 ml de soude 2 N et on extrait par de l'acétate d'éthyle. La phase organique est lavée par de la soude 2 N, de l'eau, de l'acide chlorydrique 2 N et une solution de bicarbonate de sodium N. Après séchage sur sulfate de sodium sec, le solvant est évaporé sous pression réduite pour donner une huile épaisse orangée.

Cette huile est purifiée par un passage sur colonne de gel de silice et éluée par le mélange chloroforme - 9/ méthanol - 1.

Le produit obtenu, de forme amorphe, est traité dans l'acétate d'éthyle par de l'isopropanol chlorhydrique. Après trituration du précipité dans l'éther éthylique, on isole par filtration : 24 g d'une poudre beige, insoluble dans l'eau.
F ≃ 140° C (fusion pâteuse).
Rendement = 69,8 %
Rendement total = 15,2 %.

### EXEMPLE 2

### Préparation du 2,6-diméthyl-4-(3-nitrophényl)-5-méthoxycarbonyl-1,4-dihydropyridine-3-carboxylate de N [3-(2,4,6,triméthoxybenzoyl)propyl] pipéridine-3'-yle (CRL 41780).

19,4 g (0,028 mole) de 2,6-diméthyl-4-(3-nitrophényl)-5-méthoxycarbonyl-1,4-dihydropyridine-3-carboxylate de N [3-(2,4,6,triméthoxybenzoyl)propyl] pipéridine-3-yle, chlorhydrate (CRL 41695) sont traités dans l'eau par de la soude et le précipité obtenu extrait par de l'acétate d'éthyle. Après séchage et évaporation du solvant on reprend le résidu huileux par de l'eau et on acidifie par de l'acide acétique. La solution obtenue est traitée au noir CXA, puis alcalinisée par de la soude concentrée en agitant fortement. Le précipité est isolé par filtration pour donner 15,4 g d'une poudre jaune, soluble dans l'eau en présence d'acide acétique.
F # 80° (fusion pâteuse)
Rendement = 84,5 %.

### EXEMPLE 3

### Préparation du (+)(4S, 3'S) 2,6-diméthyl-4-(3-nitrophényl)-5-méthoxycarbonyl-1,4-dihydropyridine-3-carboxylate de N-[3-(2,4,6-triméthoxybenzoyl)propyl] pipéridine-3'-yle - (CRL 41859)

### a) Préparation du 2,6-diméthyl-4-(3-nitrophényl)-1,4-dihydropyridine-3,5-dicarboxylate de diméthyle.

On chauffe au reflux pendant 5h, un mélange de 75,5 g (0,50 mole) de 3-nitrobenzaldéhyde, de 116 g (1,00 mole) d'acétoacétate de méthyle et de 55 ml (1,50 mole) d'ammoniaque à 28 % dans 150 ml de méthanol.

On isole le précipité par filtration et on purifie le gateau par un lavage à chaud dans l'éthanol absolu, pour donner 133,5 g d'une poudre jaune.
F inst (Kofler) = 208° C
Rendement = 77,2 %.

### b) Préparation du 1-éthoxyméthyl-2,6-diméthyl-4-(3-nitrophényl)-1,4-dihydropyridine-3,5-dicarboxylate de diméthyle

Au sein d'une solution de 52 g (0,150 mole du produit obtenu en (a) dans 600 ml de tétrahydrofuranne, on ajoute par fractions 7,6 g (0,157 mole) d'hydrure de sodium en suspension à 50 % dans l'huile et on agite 30 mn à la température ambiante. On introduit ensuite entre -20° et -10°C, en 15 mn une solution de 14,9 g (0,157 mole) de chlorométhyl éthyl éther dans 50 ml de tétrahydrofuranne puis on agite 1h15 vers 20°C.

Le milieu réactionnel est repris par de l'éther éthylique, lavé par de l'eau, séché sur sulfate de sodium sec et le solvant évaporé sous pression réduite. Le résidu est purifié par une cristallisation avec traitement au noir CXA dans l'éther diisopropylique pour donner 47,3 g d'une poudre beige.
F inst (Kofler) = 86° C
Rendement = 78,13 %

### c) Préparation de l'acide (+,-) 1-éthoxyméthyl-5-méthoxycarbonyl-2,6-diméthyl-4-(3-nitrophényl)-1,4-dihydropyridine-3-carboxylique

Au sein d'un solution de 18,7 g (0,813 mole) de sodium dans 212 ml de 1-diméthylamino-2-propanol on ajoute une solution de 5,4 ml (0,296 mole) d'eau dans 42 ml de 1-diméthylamino-2-propanol. En refroidissant par un bain glace/eau, on introduit en 15 mn une solution du produit obtenu en (b) dans 270 ml de benzène, on agite à la température ambiante pendant 3h et on amène à siccité sous pression réduite. Le résidu, refroidit par un bain glace/acétone, est acidifié jusqu'à pH 2 par addition d'acide chlorhydrique 3N. On extrait par du chloroforme, on lave par de l'eau, on sèche sur sulfate de sodium sec et on évapore le solvant sous pression réduite.

Le résidu est purifié par un passage sur colonne de silice et élué par le mélange benzène - acétate d'éthyle (4:1), pour donner 28,5 g d'une poudre belge.
F inst (Kofler) = 194° C
Rendement = 54,9 %.

### d) Préparation de l'acide (-) 1-éthoxyméthyl-5-méthoxycarbonyl-2,6-diméthyl-4-(3-nitrophényl)-1,4-dipydropyridine-3-carboxylique

On dissout au reflux 46 g (0,118 mole) du produit obtenu en (c) et 34,6 g (0,118 mole) de (-) cinchonidine dans 700 ml de méthanol puis on amène à siccité sous pression réduite. Le résidu est purifié par trois cristallisations successives dans l'éthanol absolu, pour donner 19,8 g d'une poudre jaune.
F inst (Kofler) = 199° C

Cette poudre est traitée par de l'acide chlorhydrique 0,1N et extraite par du chloroforme. On obtient après séchage et évaporation du solvant 12,7 g d'une poudre jaune.
F inst (Kofler) = 135° C
Rendement = 27,5 %
[α]_{D} = -18 (c = 1,78, acétone)

### e) Préparation de l'acide (-)(4R) 5-méthoxycarbonyl-2,6-diméthyl-4-(3-nitrophényl)1,4-dihydropyridine-3-carboxylique

On agite une heure à la température ambiante une solution de 14,8 g (0,0379 mole) du produit obtenu en (d) dans 450 ml d'acétone en présence de 90 ml d'acide chlorhydrique N. Le milieu réactionnel est amené à siccité sous pression réduite, extrait par de l'acétate d'éthyle puis lavé par de l'eau.

Après séchage et évaporation du solvant, le résidu est purifié par un lavage dans l'éther éthylique pour donner 6,4 g d'une poudre jaune.
Rendement = 50,9 %
[α]_{D} = - 17 (c = 0,5, acétone)

### f) Préparation de l'anhydride (-) diacétyl-d-tartrique

On chauffe lentement au reflux pendant 10 mn, une solution de 40 g (0,266 mole) d'acide D-tartrique, et 126 ml (1,333 mole) d'anhydride acétique en présence de 1,2 ml d'acide sulfurique concentré. On refroidit dans un bain glace/eau, pendant 1 h, on isole le précipité par filtration que l'on lave par du benzène.

Le gateau est purifié par un lavage dans 175 ml d'éther éthylique froid, pour donner 41,7 g d'une poudre blanche.
F inst (Kofler) = 137° C
Rendement = 72,6 %

### g) Préparation de l'acide (-) 4-chlorotartranilique

On chauffe au reflux pendant 3 h, une solution de 40 g (0,1850 mole) du produit précédent (f) et de 26 g (0,2037 mole) de 4-chloro aniline dans 370 ml de chlorure de méthylène. On extrait le milieu réactionnel par une solution de 39 g (0,592 mole) de potasse à 85 % dans 370 ml d'eau puis par 285 ml d'eau. On joint les phases aqueuses, on tiédit vers 50°C pendant 15 mn et on acidifie par de l'acide chlorhydrique concentré.

Le précipité est isolé par filtration, et le gateau purifié par une cristallisation dans le mélange eau 50/éthanol 125, pour donner 48 g d'une poudre blanche.
F # 210° C
Rendement # 100 %
[α]_{D} = -102 (c = 1,6 ; EtOH 95 %).

### h) Préparation de (-) 4-chlorotartranilate de (-) (S) 3-hydroxy pipéridine

On chauffe au reflux pour dissoudre, un mélange de 46 g (0,177 mole) du produit obtenu en g) et 35,8 g (0,3545 mole de 3-hydroxy pipéridine, dans 350 ml d'éthanol à 95 %. On laisse cristalliser à la température ambiante, on isole le précipité par filtration et on purifie le gateau par deux cristallisations succesives dans l'éthanol à 90 % pour donner 52,7 g d'aiguilles blanches.
F inst (Kofler) = 158° C
Rendement = 82,6 %
[α]_{D} = - 79 (c= 1,H₂O)

### i) Préparation de (-)(S) 3-hydroxy pipéridine

On agite à la température ambiante pendant une nuit, un mélange de 50 g (0,138 mole) du produit obtenu en h, de 19,1 g (0,138 mole) de carbonate de potassium dans 336 ml de méthanol et 144 ml de toluène. On élimine l'insoluble par filtration et on amène le filtrat à siccité sous pression réduite.

Le résidu est concrété par un lavage dans l'éther éthylique, dissout à nouveau dans le mélange méthanol-7/toluène-3, traité au noir CXA puis amené à sec pour donner 13,3 g d'une masse blanche.
Rendement = 95,4 %
[α]_{D} = -7 (c = 2, MeOH)

### j) Préparation du (-) (3S) 1-(2,4,6-triméthoxybenzoyl)-3-(3-hydroxypipéridino)propane, chlorhydrate

Au sein d'une solution au reflux de 13 g (0,1287 mole) du produit obtenu en (i) dans 35 ml de toluène, on coule en 1h une solution de 17,5 g (0,0643 mole) du 1-(2,4,6-triméthoxybenzoyl)-3-chloropropane obtenu à l'exemple 1 d dans 12 ml de toluène, on maintient le reflux 2 h et on dilue le milieu réactionnel par de l'acétate d'éthyle. On lave par de l'eau, on extrait par une solution d'acide chlorhydrique dilué, on alcalinise la phase aqueuse par de la soude concentrée et on extrait à nouveau par de l'acétate d'éthyle.

Après séchage de la phase organique sur sulfate de sodium sec, on traite par de l'isopropanol chlorhydrique.

Le précipité obtenu est purifié par une cristallisation dans l'isopropanol pour donner 11,4 g d'une poudre blanche.
F inst (Kofler) = 178° C
Rendement = 47,5 %
[α]_{D} = - 4,8 (c = 2, MeOH)

### k) Préparation du (+) (4S, 3'S) 2,6-diméthyl-4-(3-nitrophényl)-5-méthoxycarbonyl-1,4-dihydropyridine-3-carboxylate de N-[3-(2,4,6-triméthoxybenzoyl)propyl] pipéridine-3'-yle (CRL 41859)

Au sein d'une solution maintenue à -25° C de 15,6 ml de diméthylformamide et de 9,1 ml d'acétonitrile, on coule en 15 mn, une solution de 2,1 g (0,0165 mole) de chlorure d'oxalyle dans 6 ml d'acétonitrile, on agite 15 mn à -25° C et on introduit 3,9 g (0,0118 mole) du produit obtenu en (e). Après 1 h à -15° C, on refroidit à -25° C et on coule une solution de 4 g (0,0118 mole) de la base du produit obtenu en (j) dans 12,5 ml de pyridine et 2,6 ml de diméthylformamide. On agite 20 h à la température ambiante, on jette le milieu réactionnel sur 120 ml d'eau glacée et 25 ml de soude 2N et on extrait par de l'acétate d'éthyle. La phase organique est lavée par de la soude 2N, de l'eau, de l'acide chlorhydrique 2N et une solution de bicarbonate de sodium N. Après séchage sur sulfate de sodium sec, le solvant est évaporé sous pression réduite pour donner une huile épaisse orangée.

Cette huile est purifiée par un passage sur colonne de gel de silice et éluée par le mélange chloroforme/95-méthanol/5, pour donner après évaporation du solvant 6,2 g d'une poudre jaune amorphe, insoluble dans l'eau.
F # 80° C (fusion pâteuse)
Rendement = 80,7 %
[α]_{D} = + 70 (c = 1,25, MeOH)

### EXEMPLE 4

### Préparation du (-) (4R, 3'R) 2,6-diméthyl-4-(3-nitrophyényl)-5-méthoxycarbonyl-1,4-dihydropyridine-3-carboxylate de N-[3-(2,4,6-triméthoxybenzoyl)propyl] pipéridine-3'-yle (CRL 41860)

### a) Préparation de l'acide (+) 1-éthoxyméthyl-5-méthoxycarbonyl-2,6-diméthyl-4-(3-nitrophényl)-1,4-dihydropyridine-3-carboxylique

On dissout au reflux 46 g (0,118 mole) du produit obtenu à l'exemple 3(c) et 34,6 g (0,118 mole) de (+) cinchonine dans 700 ml de méthanol puis on amène à siccité sous pression réduite. Le résidu est purifié par trois cristallisations successives dans l'éthanol absolu, pour donner 19,8 g d'une poudre jaune.
F inst (Kofler) = 215° C

Cette poudre est traitée par de l'acide chlorhydrique 0,1 N et extraite par du chloroforme. On obtient après séchage et évaporation du solvant 11 g d'une poudre jaune.
F inst (Kofler) = 134° C
Rendement = 24 %
[α]_{D} = + 15 (c = 1,90, acétone)

### b) Préparation de l'acide (+)(4S) 5-méthoxycarbonyl-2,6-diméthyl-4-(3-nitrophényl)-1,4-dihydropyridine-3-carboxylique

On agite une heure à la température ambiante une solution de 13,8 g (0,035 mole) du produit obtenu en a dans 420 ml d'acétone en présence de 85 ml d'acide chlorhydrique N. Le milieu réactionnel est amené à siccité sous pression réduite, extrait par de l'acétate d'éthyle puis lavé par de l'eau.

Après séchage et évaporation du solvant, le résidu est purifié par un lavage dans l'éther éthylique pour donner 6,4 g d'une poudre jaune.
Rendement = 55 %
[α]_{D} = + 18 (c=0,5, acétone)

### c) Préparation de l'anhydride (+) diacétyl-d-tartrique

On chauffe lentement au reflux pendant 10 mn, une solution de 40 g (0,266 mole) d'acide L-tartrique, et 126 ml (1,333 mole) d'anhydride acétique en présence de 1,2 ml d'acide sulfurique concentré. On refroidit dans un bain glace/eau, pendant 1 h, on isole le précipité par filtration que l'on lave par du benzène.

Le gateau est purifié par un lavage dans 175 ml d'éther éthylique froid, pour donner 42,5 g d'une poudre blanche.
F inst (Kofler) = 136° C
Rendement = 74 %

### d) Préparation de l'acide (+) 4-chlorotartranilique

On chauffe au reflux pendant 3 h une solution de 40 g (0,1850 mole) du produit obtenu en (c) et de 26 g (0,2037 mole) de 4-chloro aniline, dans 370 ml de chlorure de méthylène. On extrait le milieu réactionnel par une solution de 39 g (0,592 mole) de potasse à 85 % dans 370 ml d'eau puis par 185 ml d'eau. On joint les phases aqueuses, on tiédit vers 50°C pendant 15 mn et on acidifie par de l'acide chlorhydrique concentré.

Le précipité est isolé par filtration, et le gateau purifié par une cristallisation dans le mélange eau 50/éthanol 125, pour donner 46,6 g d'une poudre blanche.
F # 210° C
Rendement = 97 %
[α]_{D} = + 103 (c 1,6; EtOH 95 %)

### e) Préparation de (+)(R) 4-chlorotartranilate de 3-hydroxy pipéridine

On chauffe au reflux pour dissoudre un mélange de 45 g (0,173 mole) du produit obtenu en (d) et 35 g (0,357 mole) de 3-hydroxy pipéridine, dans 350 ml d'éthanol à 95 %. On laisse cristalliser à la température ambiante, on isole le précipité par filtration et on purifie le gateau par deux cristallisations successives dans l'éthanol à 85 % pour donner 51,6 g d'aiguilles d'aiguilles blanches.
F inst (Kofler) = 158° C
Rendement = 82,7 %
[α]_{D} = + 80 (c = 1, H₂O)

### f) Préparation de (+) (R) 3-hydroxy pipéridine

On agite à la température ambiante pendant une nuit, un mélange de 50 g (0,138 mole) du produit obtenu en (e) de 19,1 g (0,138 mole) de carbonate de potassium dans 336 ml de méthanol et 144 ml de toluène. On élimine l'insoluble par filtration et on amène le filtrat à siccité sous pression réduite.

Le résidu est concrété par un lavage dans l'éther éthylique, dissout à nouveau dans le mélange méthanol-7/toluène-3, traité au noir CXA puis amené à sec pour donner 13,3 g d'une masse blanche.
Rendement = 95,4 %
[α]_{D} = + 8 (c=2, MeOH)

### g) Préparation de (+) (3R) 1-(2,4,6-triméthoxybenzoyl)-3-(3-hydroxypipéridino) propane, chlorhydrate

Au sein d'une solution au reflux de 12,7 g (0,1257 mole) du produit obtenu en (f) dans 35 ml de toluène, on coule en 1h une solution de 17,1 g (0,0628 mole) du produit 1-(2,4,6-triméthoxybenzoyl)-3-chloropropane obtenu à l'exemple 1 d dans 12 ml de toluène, on maintient le reflux 2h et on dilue le milieu réactionnel par de l'acétate d'éthyle. On lave par de l'eau, on extrait par une solution d'acide chlorhydrique dilué, on alcalinise la phase aqueuse par de la soude concentrée et on extrait à nouveau par de l'acétate d'éthyle.

Après séchage de la phase organique sur sulfate de sodium sec, on traite par de l'isopropanol chlorhydrique.

Le précipité obtenu est purifié par une cristallisation dans l'isopropanol pour donner 10,1 g d'une poudre blanche.
F inst (Kofler) = 178° C
Rendement = 47,06 %
[α]_{D} = + 5,4 (c = 2, MeOH).

### h) Préparation du (-) (4R, 3'R) 2,6-diméthyl-4-(3-nitrophényl)-5-méthoxycarbonyl-1,4-dihydropyridine-3-carboxylate de N-[3-(2,4,6-triméthoxybenzoyl)propyl] pipéridine-3'-yle (CRL 41860)

Au sein d'une solution maintenue à -25° C de 16,4 ml de diméthylformamide et de 9,6 ml d'acétonitrile, on coule en 15 mn, une solution de 2,2 g (0,0173 mole) de chlorure d'oxalyle dans 6,2 ml d'acétonitrile, on agite 15 mn à -25° C et on introduit 4,1 g (0,0124 mole) du produit obtenu en (b). Après 1h à -15° C, on refroidit à -25° C et on coule une solution de 4,2 g (0,0124 mole) de la base du produit obtenu en (g) dans 13 ml de pyridine et 2,71 ml de diméthylformamide. On agite 20 h à la température ambiante, on jette le milieu réactionnel sur 125 ml d'eau glacée et 20 ml de soude 2N et on extrait par de l'acétate d'éthyle. La phase organique est lavée par de la soude 2N, de l'eau, de l'acide chlorhydrique 2N et une solution de bicarbonate de sodium N. Après séchage sur sulfate de sodium sec, le solvant est évaporé sous pression réduite pour donner une huile épaisse orangée.

Cette huile est purifiée par un passage sur colonne de gel de silice et éluée par le mélange chloroforme/95-méthanol/5, pour donner après évaporation du solvant 4,35 g d'une poudre jaune amorphe, insoluble dans l'eau.
F # 80° C (fusion pâteuse)
Rendement = 53,9 %
[α]_{D} = - 60 (c = 1,25, MeOH)

### EXEMPLE 5

### Préparation de (+)(4R, 3'S) 2,6-diméthyl-4-(3-nitrophényl)-5-méthoxycarbonyl-1,4-dihydropyridine-3-carboxylate de N-[3-(2,4,6-triméthoxybenzoyl)propyl] pipéridine-3'-yle (CRL 41 862)

Au sein d'une solution maintenue -25° C de 18,7 ml de diméthylformamide et de 11 ml d'acétonitrile, on coule en 15 mn une solution de 2,5 g (0,0198 mole) de chlorure d'oxalyle dans 7,2 ml d'acétonitrile, on agite 15 mn à -25° C et on introduit 4,7 g (0,0142 mole) du produit obtenu à l'exemple 4b. Après 1h à-15° C, on refroidit à -25° C et on coule une solution de 4,8 g (0,0142 mole) de la base du produit obtenu à l'exemple 4j dans 15 ml de pyridine et 3,1 ml de diméthylformamide. On agite 20 h à la température ambiante, on jette le milieu réactionnel sur 130 ml d'eau glacée et 25 ml de soude 2N et on extrait par de l'acétate d'éthyle. La phase organique est lavée par de la soude 2N, de l'eau, de l'acide chlorhydrique 2N et une solution de bicarbonate de sodium N. Après séchage sur sulfate de sodium sec, le solvant est évaporé sous pression réduite pour donner une huile épaisse orangée.

Cette huile est purifiée par un passage sur colonne de gel de silice et éluée par le mélange chloroforme/95-méthanol/5, pour donner après évaporation du solvant 5,9 g d'une poudre jaune amorphe, insoluble dans l'eau.
F # 80° C (fusion pâteuse)
Rendement = 63,8 %
[α]_{D} = + 6 (c = 1,25, MeOH)

### EXEMPLE 6

### Préparation de (-)(4S, 3'R) 2,6-diméthyl-4-(3-nitrophényl)-5-méthoxycarbonyl-1,4-dihydropyridine-3-carboxylate de N-[3-(2,4,6-triméthoxybenzoyl)propyl] pipéridine-3'-yle (CRL 41863)

Au sein d'une solution maintenue à -25° C de 15,6 ml de diméthylformamide et de 9,1 ml d'acétonitrile, on coule en 15 mn une solution de 2,1 g (0,0165 mole) de chlorure d'oxalyle dans 6 ml d'acétonitrile, on agite 15 mn à -25° C et on introduit 3,9 g (0,0118 mole) du produit obtenu à l'exemple 3e. Après 1h à -15° C, on refroidit à -25° C et on coule une solution de 4g (0,0118 mole) de la base du produit obtenu à l'exemple 5g dans 12,5 ml de pyridine et 2,6 ml de diméthylformamide. On agite 20 h à la température ambiante, on jette le milieu réactionnel sur 120 ml d'eau glacée et 25 ml de soude 2N et on extrait par de l'acétate d'éthyle. La phase organique est lavée par de la soude 2N, de l'eau, de l'acide chlorhydrique 2N et une solution de bicarbonate de sodium N. Après séchage sur sulfate de sodium sec, le solvant est évaporé sous pression réduite pour donner une huile épaisse orangée.

Cette huile est purifiée par un passage sur colonne de gel de silice et éluée par le mélange chloroforme/95-méthanol/5, pour donner après évaporation du solvant 3g d'une poudre jaune amorphe, insoluble dans l'eau.
F # 80° C (fusion pâteuse)
Rendement = 39 %
[α]_{D} = -11 (c=1,25, MeOH)

### EXEMPLE 7

### Préparation de (+) (4S, 3'S) / (-) (4R, 3'R) 2,6-diméthyl-4-(3-nitrophényl)-5-méthoxycarbonyl-1,4-dihydropyridine-3-carboxylate de N-[3-(2,4,6-triméthoxybenzoyl) propyl] pipéridine-3'-yle, chlorhydrate (CRL 41 886)

On traite une solution de 20 g (0,030 mole) du composé obtenu à l'exemple 2 dans 100 ml d'acétone, par 8 ml d'isopropanol chlorhydrique. On agite et on abandonne à la température ambiante pendant 4 jours.

Le produit cristallisé est isolé par filtration puis purifié par un lavage à chaud dans l'isopropanol pour donner 8,3 g d'une poudre jaune insoluble dans l'eau.
F = 205° C
Rendement = 39,3 %

### EXEMPLE 8

### Préparation de (+) (4S, 3'R)/(-)(4R, 3'S) 2,6-diméthyl-4-(3-nitrophényl)-5-méthoxycarbonyl-1,4-dihydropyridine-3-carboxylate de N-[3-(2,4,6-triméthoxybenzyl)propyl] pipéridine-3'-yle, chlorhydrate (CRL 41887)

On traite une solution de 20 g (0,030 mole) du composé obtenu à l'exemple 2 dans 100 ml d'acétone, par 8 ml d'isopropanol chlorhydrique. On agite et on abandonne à la température ambiante pendant 4 jours.

Le produit cristallisé est écarté par filtration, le filtrat après traitement au noir CXA, amené à siccité sous pression réduite et le résidu gommeux concrété par deux lavages dans l'acétate d'éthyle.

Le produit cristallisé obtenu est purifié par un lavage à chaud dans l'isopropanol pour donner 3,5 g d'une poudre jaune, insoluble dans l'eau.
F = 212°
Rendement = 24,6 %.

### EXEMPLE 9

### Préparation du 2,6-diméthyl-4-(3-nitrophényl)-5-méthoxycarbonyl-1,4-dihydropyridine-3-carboxylate de N [3-(2,4,6-triméthoxybenzoyl)propyl] pipéridine-3'-yle, lactate (CRL 41 813)

On mélange dans 150 ml d'eau distillée, 10 g (0,0153 mole) du composé obtenu à l'exemple 2 et 1,4 g (0,0153 mole) d'acide lactique.

On agite une nuit à température ambiante, on traite la solution au noir CXA et on amène à siccité sous pression réduite.

On obtient 9 g d'une poudre jaune amorphe, soluble dans l'eau froids mais qui précipite au chauffage.
Fusion pâteuse
Rendement = 86,75 %.

### EXEMPLE 10

### Préparation du 2,6-diméthyl-4-(3-nitrophényl)-5-méthoxycarbonyl-1,4-dihydropyridine-3-carboxylate de N [3-(2,4,6-triméthoxybenzoyl) propyl] pipéridine-3'-yle, glutamate (CRL 41 814)

On mélange 9 g (0,0138 mole) du composé obtenu à l'exemple 1 et 2,3 g (0,0138 mole) d'acide glutamique hydratée dans 50 ml de méthanol. On agite à la température ambiante et on amène la solution obtenue à siccité sous pression réduite.

On isole 11 g d'une poudre jaune amorphe, insoluble dans l'eau.
Fusion pâteuse
Rendement = 99 %.

### EXEMPLE 11

### Préparation du 2,6-diméthyl-4-(3-nitrophényl)-5 méthoxycarbonyl-1,4-dihydropyridine-3-carboxylate de N [3-(2,4,6-triméthoxybenzoyl)propyl] pipéridine-3'-yle, aspartate (CRL 41 815)

On mélange 10 g (0,0153 mole) du composé obtenu à l'exemple 2 et 2,05 g (0,0153 mole) d'acide aspartique dans 100 ml de méthanol et 150 ml d'eau distillée. On agite à la température ambiante et on évapore la solution obtenue sous pression réduite pour donner 10,5 g d'une poudre jaune amorphe, insoluble dans l'eau.
Fusion pâteuse
Rendement = 87,5 %.

On donnera ci-après des résultats toxicologiques et pharmacologiques mettant en évidence les propriétés des composés selon l'invention.

### 1) Toxicité aiguë

La toxicité aiguë du composé de l'exemple 1 a été déterminée par voie intrapéritonéale chez le rat (rats Sprague Dawley), le produit étant administré en suspension aqueuse à 20 mg/ml.
DL₅₀ = 249 ± 50 mg/kg

A titre de comparaison, le DL₅₀ de la nicardipine déterminée dans les mêmes conditions est de 233 ± 65 mg/kg.

### 2) Activité anticalcique

On a déterminé l'activité anticalcique in vitro sur le modèle de la contraction de l'aorte isolée de rat par du KCl (voir Bernard Swynghedauw - Expérimentation animale en cardiologie, coll. INSERM Medecine Sciences Flammarion).

A cet effet on a, dans un premier temps, déterminé les CI₅₀ au niveau du récepteur (sur le site DHP membranaire avec le radioligand ³H isradipine) et on a déterminé avec ces doses le pourcentage d'inhibition dans la contraction de l'aorte isolée.

Les résultats sont les suivants :

| Composé testé Exemple | Dose (M) | % inhibition |
|---|---|---|
| 2 | 10⁻⁸ | 60 |
| 3 | 3.10⁻⁹ | 70 |
| 6 | 2.10⁻⁹ | 80 |
| 7 | 4.10⁻⁹ | 40 |
| 8 | 6.10⁻¹⁰ | 60 |

### 3) Effets sur la pression artérielle moyenne et la fréquence cardiaque.

Les effets ont été recherchés chez le rat Wistar génétiquement hypertendu éveillé (6 rats/composé). Les composés ont été administrés par voie orale en suspension à la dose de 16 mg/kg (sous 5,3 ml/kg).

Les résultats sont les suivants :

| Pression artérielle | CRL 41695 | Nicardipine |
|---|---|---|
| Variation au bout de 3 h | - 50 % | - 39 % |
| Variation au bout de 7 h | - 42 % | - 27 % |

| Fréquence cardiaque | CRL 41695 | Nicardipine |
|---|---|---|
| Variation au bout de 5 mn | + 13 % | + 32 % |
| Variation au bout de 5 h | 0 % | + 23 % |

On observe avec le composé de l'Exemple 1 une hypotension nettement plus forte pendant au moins 7 heures. La fréquence cardiaque n'est que légèrement augmentée par le composé de l'exemple 1, alors qu'après 5 mn la nicardipine provoque un net effet tachycardisant.

On observe d'ailleurs avec la nicardipine un effet tachycardisant secondaire qui atteint son maximum au bout de 5 h. Un tel effet tachycardisant n'est pas observé avec le composé de l'exemple 1.

### 4) Effet sur l'hyperperméabilité à l'histamine.

L'effet du CRL 41695 par voie i.p. sur l'hyperperméabilité à l'histamine a été recherché sur le rat Sprague Dawley.

Le paramètre étudié est l'effet sur l'extravasation intradermique de Bleu d'Evans induite par injection d'histamine.

24 h après tonte et épilation des flancs de l'animal, on injecte le CRL 41695 à la dose de 10 mg/kg (en dispersion dans NaCl 0,9 % + carboxyméthylcellulose à 5 %) par voie i.p. et 10 mn après 0,25 ml/100 g de Bleu d'Evans à 2 % par voie i.v.

On injecte ensuite par voie intradermique 0,1 ml de NaCl 0,9 % dans le flanc gauche et 0,1 ml d'histamine (50 µg) dans le flanc droit.

40 mn après on sacrifie les animaux et on prelève les zones d'injection, puis l'on extrait le Bleu d'Evans de la peau dans du formamide à 65 %. On élimine le solvant par filtration et on détermine le densité optique à 620 nm.

Par rapport au témoin (Solution NaCl) on observe une diminution de la concentration intradermique de Bleu d'Evans de 50 %.

Le composé de l'exemple 1 diminue donc très fortement, à la dose de 10 mg/kg i.p., la concentration intradermique de Bleu d'Evans induite par l'histamine et présente donc un effet anti hyperperméabilisant.

La présente invention a également pour objet des compositions thérapeutiques comprenant à titre de principe actif un composé selon l'invention ou l'un de ses sels d'addition avec des acides pharmaceutiquement acceptables.

Les compositions thérapeutiques selon l'invention peuvent être administrées à l'homme ou aux animaux par voie orale ou parentérale.

Elles peuvent être sous la forme de préparations solides, semi-solides ou liquides. Comme exemple, on peut citer les comprimés, les gélules, les suppositoires, les solutions ou suspensions injectables, ainsi que les formes-retard et les formes implantées à libération lente.

Dans ces compositions, le principe actif est généralement mélangé avec un ou plusieurs excipients pharmaceutiquement acceptables habituels bien connus de l'homme de l'art.

La quantité de principe actif administrée dépend évidemment du patient qui est traité, de la voie d'administration et de la sévérité de la maladie.

En raison notamment de ses propriétés sur l'hyperperméabilité, les compositions selon l'invention trouvent en particulier une application dans le traitement des oedèmes périphériques et dans la protection tissulaire rénale des insuffisants rénaux et diabétiques.

## Revendications

1. Composé qui est le 2,6-diméthyl-4-(3-nitrophényl)-5-méthoxycarbonyl-1,4-dihydropyridine-3-carboxylate de N[3-(2,4,6-triméthoxybenzoyl)propyl] pipéridine-3'-yle, et ses sels d'addition avec des acides pharmaceutiquement acceptables.

2. Composé selon la revendication 1, qui est le (+)(4S, 3'S)2,6-diméthyl-4-(3-nitrophényl)-5-méthoxycarbonyl-1,4-dihydropyridine-3-carboxylate de N-[3-(2,4,6-triméthoxybenzoyl)propyl]pipéridine-3'-yle et ses sels d'addition avec des acides pharmaceutiquement acceptables.

3. Composé selon la revendication 1, qui est le (-)(4S, 3'R)2,6-diméthyl-4-(3-nitrophényl)-5-méthoxycarbonyl-1,4-dihydropyridine-3-carboxylate de N-[3-(2,4,6-triméthoxybenzoyl)propyl]pipéridine-3'-yle et ses sels d'addition avec des acides pharamceutiquement acceptables.

4. Procédé de préparation d'un composé selon la revendication 1, caractérisé en ce que l'on fait réagir sur un acide de formule 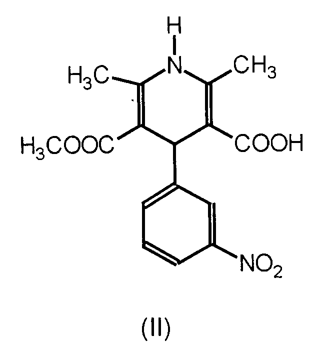 un alcool de formule

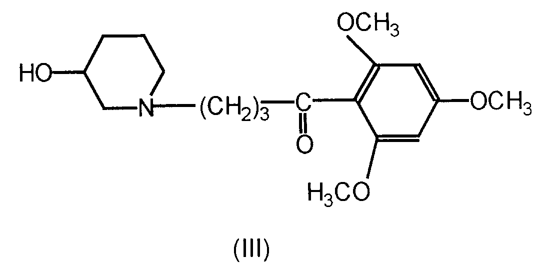

5. Composition thérapeutique comprenant à titre de principe actif un composé selon l'une quelconque des revendications 1 à 3.

## Claims

1. Compound which is N-[3-(2,4,6-trimethoxybenzoyl)-propyl]-piperidin-3'-yl 2,6-dimethyl-4-(3-nitrophenyl)-5-methoxycarbonyl-1,4-dihydropyridine-3-carboxylate, and the pharmaceutically acceptable acid addition salts thereof.

2. Compound according to claim 1, which is N-[3-(2,4,6-trimethoxybenzoyl)-propyl]-piperidin-3'-yl (+)(4S,3'S)2,6-dimethyl-4-(3-nitrophenyl)-5-methoxycarbonyl-1,4-dihydropyridine-3-carboxylate and the pharmaceutically acceptable acid addition salts thereof.

3. Compound according to claim 1, which is N-[3-(2,4,6-trimethoxybenzoyl)-propyl]-piperidin-3'-yl (-)(4S,3'R)2,6-dimethyl-4-(3-nitrophenyl)-5-methoxycarbonyl-1,4-dihydropyridine-3-carboxylate and the pharmaceutically acceptable acid addition salts thereof.

4. Process for preparing a compound according to claim 1, characterised in that an acid of formula 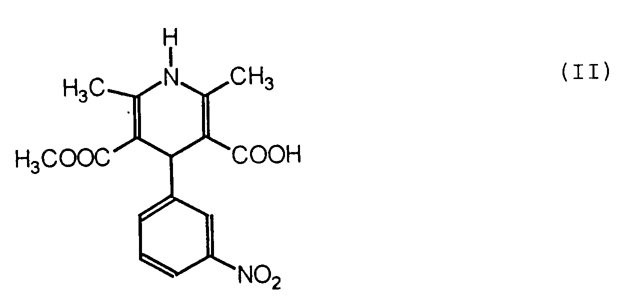 is reacted with an alcohol of formula

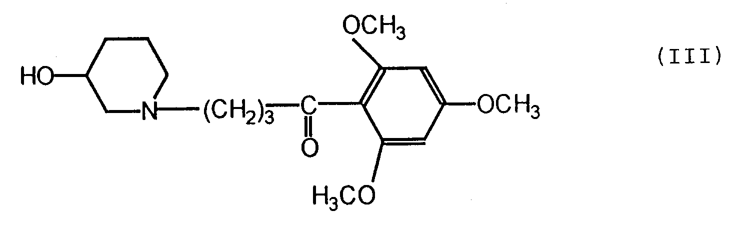

5. Therapeutic composition containing as active principle a compound according to any one of claims 1 to 3.

## Patentansprüche

1. Verbindung, nämlich 2,6-Dimethyl-4-(3-nitrophenyl)-5-methoxycarbonyl-1,4-dihydropyridin-3-carbonsäure-N-[3-(2,4,6-trimethoxybenzoyl)-propyl-piperidin-3'-yl-ester und dessen Additionssalze mit pharmazeutisch annehmbaren Säuren.

2. Verbindung nach Anspruch 1, nämlich (+)-(4S,3'S)-2,6-dimethyl-4-(3-nitrophenyl)-5-methoxycarbonyl-1,4-dihydropyridin-3-carbonsäure-N-[3-(2,4,6-trimethoxybenzoyl)-propyl]-piperidin-3'-yl-ester und dessen Additionssalze mit pharmazeutisch annehmbaren Säuren.

3. Verbindung nach Anspruch 1, nämlich (-)-(4S,3'R)-2,6-dimethyl-4-(3-nitrophenyl)-5-methoxycarbonyl-1,4-dihydropyridin-3-carbonsäure-N-[3-(2,4,6-trimethoxybenzoyl)-propyl]-piperidin-3'-yl-ester und dessen Additionssalze mit pharmazeutisch annehmbaren Säuren.

4. Verfahren zur Herstellung einer Verbindung nach Anspruch 1, dadurch gekennzeichnet, daß man eine Säure der Formel 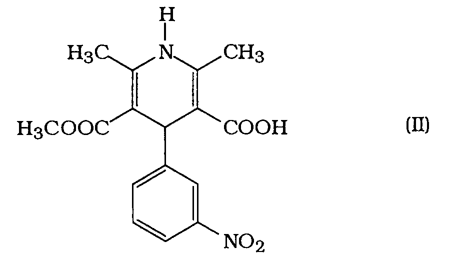 mit einem Alkohol der Formel 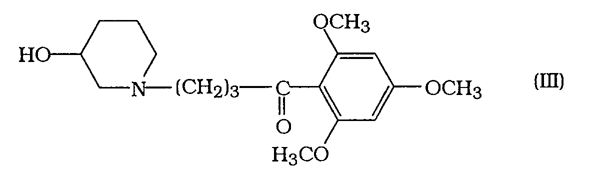 umsetzt.

5. Therapeutische Zubereitung enthaltend als Wirkstoff eine Verbindung nach einem der Ansprüche 1 bis 3.
